# EUROPEAN PATENT SPECIFICATION

(11) **EP 0 491 865 B1**
(45) Date of publication and mention of the grant of the patent: **11.12.1996**
(21) Application number: 90915038.5
(22) Date of filing: 14.09.1990
(51) Int. Cl.: A61K 39/09, C12N 15/31, C07K 16/46

(54) **CONJUGATE VACCINE FOR GROUP B STREPTOCOCCUS**
KONJUGATIMPFSTOFF FÜR GRUPPE B-STREPTOCOCCUS
VACCIN CONJUGUE POUR STREPTOCOQUE DU GROUPE B

(30) Priority: 15.09.1989 US 408036
(43) Date of publication of application: 01.07.1992
(73) Proprietor: THE GENERAL HOSPITAL CORPORATION, Boston, MA 02114 (US); BRIGHAM AND WOMEN'S HOSPITAL, Boston, Massachusetts 02115 (US)
(72) Inventor: MICHEL, James, L., Waban, MA 02168 (US); KASPER, Dennis, L., Newton Centre, MA 02159 (US); AUSUBEL, Frederick, M., Newton, MA 02161 (US)
(74) Representative: Aulmich, Gerhard, Dr.
(86) International application number: US9005251
(87) International publication number: WO9104049

(56) References cited:
- WO-A-89/00583
- US-A- 4 356 170
- US-A- 4 367 222
- US-A- 4 830 852
- NEW ENGLAND JOURNAL OF MEDICINE, vol. 319, no. 18, 03 November 1988; BAKER et al., pp. 1180-1185
- INFECTION & IMMUNITY, vol. 57, no. 10, October 1989; KUYPERS et al., pp. 3058- 3065

## Description

### FIELD OF THE INVENTION:

The invention relates to the fields of microbiology and vaccine technology, and concerns the development of a vaccine capable of conferring immunity to infection by group B Streptococcus.

### BACKGROUND OF THE INVENTION:

Bacteria of the Streptococcus genus have been implicated as causal agents of disease in humans and animals. The Streptococci have been divided into immunological groups based upon the presence of specific carbohydrate antigens on their cell surfaces. At present, groups A through O are recognized (Davis, B.D. et al., In: Microbiology, 3nd. Edition, page 609, (Harper & Row, 1980). Streptococci are among the most common and important bacteria causing human disease.

Although Streptococci of the B group are associated with animal disease (such as mastitis in cattle), Streptococcus agalactiae (a group B Streptococci) has emerged as the most common cause of human neonatal sepsis in the United States and is thought to be responsible for over 6000 deaths annually (Hill, H.R. et al., Sexually Transmitted Diseases, McGraw Hill, pp. 397-407). Group B Streptococcus is also an important pathogen in late-onset meningitis in infants, in postpartum endometritis, and in infections in immunocompromised adults (Patterson, M.J. et al., Bact. Rev. 40:774-792 (1976)). Although the organism is sensitive to antibiotics, the high attack rate and rapid onset of sepsis in neonates and meningitis in infants results in both high morbidity (50%) and mortality (20%) (Baker, C.J. et al., New Eng. J. Med. (Editorial) 314(26):1702-1704 (1986); Baker, C.J. et al., J. Infect. Dis. 136:137-152 (1977)).

Group B Streptococcus is a common component of normal human vaginal and colonic flora. While the most common route of neonatal infection is intrapartum from vaginal colonization, nosocomial spread in newborn nurseries has also been described (Patterson, M.J. et al., Bact. Rev. 40:774-792 (1976)). However, only a small percentage of infants colonized with group B Streptococcus develop serious infections. The role of both host factors and bacterial virulence determinants in the transition from colonization to infection is not well understood.

Several proteins from group B Streptococcus are throught to have a role in virulence and immunity (Ferrieri, P., Rev. Infect. Dis. 10:S363 (1988)). In 1975, Lancefield defined the C proteins of group B Streptococcus by their ability to elicit protective immunity (Lancefield, R.C., et al., J. Exp. Med. 142:165-179 (1975)). This group of proteins is thought to contain several different polypeptides and antigenic determinants. In view of these findings, efforts to prevent infections with group B Streptococcus have been directed towards the use of prophylactic antibiotics and the development of a vaccine against group B Streptococcus (Baker, C.J., et al., Rev. of Infec. Dis. 7:458-467 (1985), Baker, C.J. et al., New Eng. J. Med. (Editorial) 314(26):1702-1704 (1986)). Polysaccharide vaccines against group B Streptococcus are described by Kasper, D.L. (U.S. Patent 4,207,414 and U.S. Reissue Patent RE31672, and U.S. Patent Nos. 4,324,887, 4,356,263, 4,367,221, 4,367,222, and 4,367,223), by Carlo, D.J. (U.S. Patent 4,413,057, European Patent Publication 38,265), and by Yavordios, D. et al. (European Patent Publication 71,515).

Except for the small sub-population of infants in whom both maternal colonization with group B Streptococcus and other perinatal risk factors can be identified, the use of prophylactic antibiotics has not been practical or efficacious in preventing the majority of cases (Boyer, K.M., et al., New Eng. J. Med. 314(26):1665-1669 (1986)). Intrapartum chemoprophylaxis has not gained wide acceptance for the following reasons: (1) It has not been possible to identify maternal colonization by group B Streptococcus in a fast, reliable and cost-effective manner; (2) About 40% of neonatal cases occur in low-risk settings; (3) It has not been considered practical to screen and/or treat all mothers or infants who are potentially at risk; and (4) antibiotic prophylaxis has not appeared to be feasible in preventing late-onset meningitis (7200 cases per year in the United States) or postpartum endometritis (45,000 cases annually) (Baker, C.J. et al., New Eng. J. Med. (Editorial) 314:1702-1704 (1986)).

In the international application WO 87/06267 antigens and vaccines containing purified oligomers (1-50 units) of the repeating pentasaccharide unit of type III Group B Streptococcus (III GBS) polysaccharide capsule, methods of making the antigen by recovering polysaccharide from cultured III GBS or medium and digesting the polysaccharide with a specific endo-β-galactosidase, enzymatic cleavage of bacterial polysaccharide to make purified oligomers, a purified trypsin-resistant C surface protein, m.w. about 14.000 and vaccine, passive immunization using the above vaccines, immunoassays for GBS immunodeterminants or anti-GBS antibodiesare described.

In US-patent No. 4,830,852 covalently-modified neutral bacterial polysaccharides; covalent conjugates of such polysaccharides linked by a bigeneric spacer, with immunogenic bacterial membrane or other proteins, which conjugates are useful components of bacterial vaccines; and methods of preparing such polysaccharides and conjugates are described.

In US-patent No. 4,673,574 an immunogenic conjugate which is the reductive amination product of an immunogenic capsular polymer fragment having a reducing end and derived from a bacterial capsular polymer of a bacterial pathogen, and a bacterial toxin or toxoid are described.

In US-patent No. 4,356,170 antigen polysaccharides are modified to generate a terminally-located aldehyde group by controlled oxidation of vicinal hydroxyl groups, e.g. of unlinked terminal non-reducing sialic acid residues. In some cases where there is a reducing end group, e.g. of the type N-acetylmannosamine residue, it can be made into the most susceptible site for oxidation by initially reducing it to its open chain hydroxyl form, e.g. N-acetylmannosaminitol. The vicinal hydroxyl oxidation is controlled to yield a reactive aldehyde group which is then covalently linked to a free amino group of a selected protein by reductive amination. The resulting polysaccharide-protein conjugates are soluble and have been found to have enhanced antigenicity compared to the polysaccharide alone. This terminal aldehyde:free amine group reductive amination can be applied to various polysaccharide antigens and various well-tolerated proteins, preferably protein immunogens. For example, meningococcal group A, B and C polysaccharides have been linked to tetanus toxoid to give soluble conjugates which have been found to have advantageous immunogenic properties.

### DEPOSIT OF MICROORGANISMS:

Plasmids pJMS1 and pJMS23 are derivatives of plasmid pUX12 which contain DNA capable of encoding antigenic Streptococci proteins that may be used in accordance with the present invention. Plasmid pUX12 is a derivative of plasmid pUC12. Plasmids pJMS1 and pJM223 were deposited on September 15, 1989, at the American Type Culture Collection, Rockville, MD. and given the designations ATCC 40659 and ATCC 40660, respectively.

### SUMMARY OF THE INVENTION:

Streptococcus agalactiae is the most common cause of neonatal sepsis in the United States and is responsible for between 6,000 and 10,000 deaths per year. While the type-specific polysaccharide capsule of group B Streptococcus is immunogenic and carries important protective antigens, clinical trials of a polysaccharide vaccine have shown a poor response rate (Baker, C.J. et al., New Engl. J. Med. 319:1180 (1980); Insel, R.A., et al., New Eng. J. Med. (Editorial) 319(18):1219-1220 (1988)).

The present invention concerns the development of a conjugate vaccine to group B Streptococcus, (i.e. Streptococcus agalactiae) that utilizes to a protective protein antigen expressed from a gene cloned from group B Streptococcus. This novel conjugate vaccine has the advantages both of eliciting T-cell dependent protection via the adjuvant action of the carrier protein and also providing additional protective epitopes that are present on the cloned group B Streptococcus protein (Insel, R.A., et al., New Eng. J. Med. (Editorial) 319(18):1219-1220 (1988); Baker, C.J., et al., Rev. of Infec. Dis. 7:458-467 (1985)).

In detail, the invention provides a conjugate vaccine capable of conferring host immunity to an infection by group B Streptococcus which comprises (a) a polysaccharide conjugated to (b) a protein; wherein both the polysaccharide and the protein are characteristic molecules of the group B Streptococcus.

The invention also concerns a method for preventing or attenuating an infection caused by a group B Streptococcus which comprises administering to an individual, suspected of being at risk for such an infection, an effective amount of a conjugate vaccine capable of conferring host immunity to the infection, the vaccine comprising: (a) a polysaccharide conjugated to (b) a protein; wherein both the polysaccharide and the protein are characteristic molecules of the group B Streptococcus.

The invention further concerns a method for preventing or attenuating infection caused by a group B Streptococcus which comprises administering to a pregnant female an effective amount of a conjugate vaccine capable of conferring immunity to the infection to an unborn offspring of the female, the vaccine comprising: (a) a polysaccharide conjugated to (b) a protein; wherein both the polysaccharide and the protein are characteristic molecules of the group B Streptococcus.

The invention also provides a method for preventing or attenuating an infection caused by a group B Streptococcus which comprises administering to an individual suspected of being at risk for such an infection an effective amount of an antisera elicited from the exposure of a second individual to a conjugate vaccine capable of conferring host immunity to the infection, the vaccine comprising: (a) a polysaccharide conjugated to (b) a protein; wherein both the polysaccharide and the protein are characteristic molecules of the group B Streptococcus.

### BRIEF DESCRIPTION OF THE FIGURES:

Figure 1 shows the modifications of pUC12 to create the plasmid pUX12.

Figure 2 shows the restriction and transcriptional map of the plasmid pUX12.

Figure 3 shows the modifications which were made to pUX12 in order to produce the +1 reading frame plasmid pUX12+1 (A), and which produce the -1 reading frame plasmid pUX12-1 (C). (B) shows a construction which is additionally capable of resulting in a -1 reading frame plasmid.

Figure 4 shows the result of mouse protection studies employing rabbit antisera against S1 and S23. Protection was observed in mice inoculated with anti-S1 antisera (p<0.002) or with anti-S23 antisera (p<0.022). Due to the sample size used, this difference in the observed statistical significicance between the S1 and S23 experiments is not significant. In the Figure, the mice surviving per total tested is reported as a fraction above each bar.

### DESCRIPTION OF THE PREFERRED EMBODIMENTS:

### Significance and Clinical Perspective

Maternal immunoprophylaxis with a vaccine to group B Streptococcus has been proposed as a potential route for protecting against infection both in the mother and in the young infant through the peripartum transfer of antibodies (Baker, C.J. et al., New Eng. J. Med. (Editorial) 314(26):1702-1704 (1986); Baker, C.J. et al., New Eng. J. Med. 319:1180 (1988); Baker, C.J. et al., J. Infect. Dis. 7:458 (1985)). As is the case with other encapsulated bacteria, susceptibility to infection correlates with the absence of type-specific antibody (Kasper, D.L., et al., J. Clin, Invest. 72:260-269 (1983), Kasper, D.L., et al., Antibiot. Chemother. 35:90-100 (1985)). The lack of opsonically active type-specific anti-capsular antibodies to group B Streptococcus is a risk factor for the development of disease following colonization with group B Streptococcus (Kasper, D.L. et al., J. Infec. Dis. 153:407-415 (1986)).

One approach has been to vaccinate with purified type-specific capsular polysaccharides. Methods of producing such vaccines, and the use of such vaccines to immunize against group B Streptococcus are disclosed by Kasper, D.L. (U.S. Patent 4,207,414 and U.S. Reissue Patent RE31672, and U.S. Patent Nos. 4,324,887, 4,356,263, 4,367,221, 4,367,222, and 4,367,223), by Carlo, D.J. (U.S. Patent 4,413,057, European Patent Publication 38,265), and by Yavordios, D. et al. (European Patent Publication 71,515).

Although the polysaccharide capsule of group B Streptococcus is well characterized and has been shown to play a role in both virulence and immunity (Kasper, D.L. J. infect. Dis. 153:407 (1986)), these capsular components have been found to vary in their immunogenicity depending both on the specific capsular type and on factors in the host's immune system (Baker, C.J., et al., Rev. of Infec. Dis. 7:458-467 (1985)). A recently completed clinical trial evaluating a capsular polysaccharide vaccine of group B Streptococcus showed an overall response rate of 63% and indicated that such a vaccine was not optimally immunogenic (Baker C.J., et al., New Eng. J. Med. 319(18):1180-1185 (1988)).

Differences in immunogenicity have also been observed with the capsular polysaccharides of other bacteria. For example, the vaccine against the type C meningococcal capsule is highly active while the group B meningococcal polysaccharide vaccine is not immunogenic (Kasper, D.L. et al., J. Infec. Dis. 153:407-415 (1986)). T-cell independent functions of the host's immune system are often required for mounting an antibody response to polysaccharide antigens. The lack of a T-cell independent response to polysaccharide antigens may be responsible for the low levels of antibody against group B Streptococcus present in mothers whose children subsequently develop an infection with group B Streptococcus. In addition, children prior to 18 or 24 months of age have a poorly developed immune response to T-cell independent antigens.

### Determinants of Virulence and Immunity in group B Streptococcus

There are five serotypes of group B Streptococcus that share a common group specific polysaccharide antigen. However, antibody of the group antigen is not protective in animal models. Lancefield originally classified group B Streptococcus into four serotypes (Ia, Ib, II and III) using precipitin techniques. The composition and structure of the unique type-specific capsular polysaccharides for each of the serotypes was subsequently determined (Jennings, H.J., et al., Biochem. 22:1258-1264 (1983), Kasper, D.L. et al., J. Infec. Dis. 153:407-415 (1986), Wessels, M.R., et al., Trans. Assoc. Amer. Phys. 98:384-391 (1985)). Wilkinson defined a fifth serotype, Ic, by the identification of a protein antigen (originally called the Ibc protein) present on all strains of serotype Ib and some strains with the type Ia capsule (Wilkinson, H.W., et al., J. Bacteriol. 97:629-634 (1969), Wilkinson, H.W., et al., Infec. and Immun. 4:596-604 (1971)). This protein was later found to vary in prevalence between the different serotypes of group B Streptococcus but was absent in serotype Ia (Johnson, D.R.. et al., J. Clin. Microbiol. 19:506-510 (1984)).

The nomenclature has recently been changed to classify the serotypes of group B Streptococcus solely by the capsular type-specific polysaccharides, and a fifth capsular type has also been described (type IV) (Pritchard, D.G., et al., Rev. Infec. Dis. 10(8):5367-5371 (1988)). Therefore, the typing of group B Streptococcus strains is no longer based on the antigenic Ibc protein, which is now called the C protein. The type Ic strain is reclassified as serotype Ia on the basis of its capsular polysaccharide composition, with the additional information that it also carries the C protein.

Immunological, epidemiological and genetic data suggest that the type-specific capsule plays an important role in immunity to group B Streptococcus infections. The composition and structure of the type-specific capsular polysaccharides and their role in virulence and immunity have been the subjects of intensive investigation (Ferrieri, P. et al., Infec. Immun. 27:1023-1032 (1980), Krause, R.M., et al., J. Exp. Med. 142:165-179 (1975), Levy, N.J., et al., J. Infec. Dis. 149:851-860 (1984), Wagner, B., et al., J. Gen. Microbiol. 118:95-105 (1980, Wessels, M.R., et al., Trans. Assoc.Amer. Phys. 98:384-391 (1985)).

Controversy has existed regarding the structural arrangement of the type-specific and group B streptococcal polysaccharides on the cell surface, on the immunologically important determinants with in the type-specific polysaccharide, and on the mechanisms of capsule determined virulence of group B Streptococcus (Kasper, D.L. et al., J. Infec. Dis. 153:407-415 (1986)). To study the role of the capsule in virulence, Rubens et al. used transposon mutagenesis to create an isogeneic strain of type III group B Streptococcus that is unencapsulated (Rubens, C.E., et al., Proc. Natl. Acad. Sci. USA 84:7208-7212 (1987)). They demonstrated that the loss of capsule expression results in significant loss of virulence in a neonatal rat model. However, the virulence of clinical isolates with similar capsular composition varies widely. This suggests that other bacterial virulence factors, in addition to capsule, play a role in the pathogenesis of group B Streptococcus.

A number of proteins and other bacterial products have been described in group B Streptococcus whose roles in virulence and immunity have not been established, CAMP (Christine Atkins-Much Peterson) factor, pigment (probably carotenoid), R antigen, X antigen, anti-phagocytic factors and poorly defined "pulmonary toxins" (Ferrieri, P., et al., J. Exp. Med. 151:56-68 (1980), Ferrieri, P. et al., Rev. Inf. Dis. 10(2):1004-1071 (1988), Hill, H.R. et al., Sexually Transmitted Diseases, McGraw-Hill, pp. 397-407). The C proteins are discussed below.

Isogeneic strains of group B Streptococcus lacking hemolysin show no decrease in virulence in the neonatal rat model (Weiser, J.N., et al., Infec. and Immun. 55:2314-2316 (1987)). Both hemolysin and neuraminidase are not always present in clinical isolates associated with infection. The CAMP factor is an extracellular protein of group B Streptococcus with a molecule weight of 23,500 daltons that in the presence of staphylococcal beta-toxin (a sphingomyelinase) leads to the lysis of erythrocyte membranes. The gene for the CAMP factor in group B Streptococcus was recently cloned and expressed in E. coli (Schneewind, O., et al., Infec. and Immun. 56:2174-2179 (1988)). The role, if any, of the CAMP factor, X and R antigens, and other factors listed above in the pathogenesis of group B Streptococcus is not disclosed in the prior art (Fehrenbach, F.J., et al., In: Bacterial Protein Toxins, Gustav Fischer Verlag, Stuttgart (1988); Hill, H.R. et al., Sexually Transmitted Diseases, McGraw-Hill, NY, pp. 397-407 (1984)).

The C protein(s) are a group of a cell surface associated protein antigens of group B Streptococcus that were originally extracted from group B Streptococcus by Wilkinson et al. (Wilkinson, H.W., et al., J. Bacteriol. 97:629-634 (1969), Wilkinson, H.W., et al., Infec. and Immun. 4:596-604 (1971)). They used hot hydrochloric acid (HCl) to extract the cell wall and trichloroacetic acid (TCA) to precipitate protein antigens. Two antigenically distinct populations of C proteins have been described: (1) A group of proteins that are sensitive to degradation by pepsin but not by trypsin, and called either TR (trypsin resistant) or α. (2) Another group of group B Streptococcus proteins that are sensitive to degradation by both pepsin and trypsin, and called TS (trypsin sensitive) or β (Bevanger, L., et al., Acta Path. Microbio. Scand Sect. B. 87:51-54 (1979), Bevanger, L., et al., Acta Path. Microbio. Scand. Sect. B. 89:205-209 (1981), Bevanger, L. et al., Acta Path. Microbio. Scand. Sect. B. 91:231-234 (1983), Bevanger, L. et al., Acta Path. Microbio. Scand. Sect. B. 93:113-119 (1985), Bevanger, L., et al., Acta Path Microbiol. Immunol. Scand. Sect. B. 93:121-124 (1985), Johnson, D.R., et al., J. Clin. Microbiol. 19:506-510 (1984), Russell-Jones, G.J., et al., J. Exp. Med. 160:1476-1484 (1984)).

In 1975, Lancefield et al. used mouse protection studies with antisera raised in rabbits to define the C proteins functionally for their ability to confer protective immunity against group B Streptococcus strains carrying similar protein antigens (Lancefield, R.C., et al., J. Exp. Med. 142:165-179 (1975)). Numerous investigators have obtained crude preparations of antigenic proteins from group B Streptococcus, that have been called C proteins, by chemical extraction from the cell wall using either HCl or detergents (Bevanger, L., et al., Acta Path. Microbio. Scand. Sect. B. 89:205-209 (1981), Bevanger, L. et al., Acta Path. Microbio. Scand. Sect. B. 93:113-119 (1985), Russell-Jones, G.J., et al., J. Exp Med. 160:1476-1484 (1984), Valtonen, M.V., et al., Microb. Path. 1:191-204 (1986), Wilkinson, H.W., et al., Infec. and Immun. 4:596-604 (1971)). The reported sizes for these antigens have varied between 10 and 190 kilodaltons, and a single protein species has not been isolated or characterized (Ferrieri, P. et al., Rev. Inf. Dis. 10(2):1004-1071 (1988)).

By screening with protective antisera, C proteins can be detected in about 60% of clinical isolates of group B Streptococcus, and are found in all serotypes but with differing frequencies (Johnson, D.R., et al., J. Clin Microbiol. 19:506-510 (1984)). Individual group B Streptococcus isolates may have both the TR and TS antigens, or only one, or neither of these antigens. Except for the ability of the partially purified antigens to elicit protective immunity, the role of these antigens in pathogenesis has not been studied in vitro. In vivo studies with group B Streptococcus strains that carry C proteins provides some evidence that the C proteins may be responsible for resistance to opsonization (Payne, N.R., et al., J. Infec. Dis. 151:672-681 (1985)), and the C proteins may inhibit the intracellular killing of group B Streptococcus following phagocytosis (Payne, N.R., et al., Infect. and Immun. 55:1243-1251 (1987)). It has been shown that type II strains of group B Streptococcus carrying the C proteins are more virulent in the neonatal rat sepsis model (Ferrieri, P., et al., Infect. Immun. 27:1023-1032 (1980), Ferrieri, P. et al., Rev. Inf. Dis. 10(2):1004-1071 (1988)). Since there is no genetic data on the C proteins, isogeneic strains lacking the C proteins have not previously been studied. There is evidence that one of the TS, or β, C proteins binds to IgA (Russell-Jones, G.J., et al., J. Exp Med. 160:1476-1484 (1984)). The role, if any, that the binding of IgA by the C proteins has on virulence is, however, not disclosed.

In 1986, Valtonen et al. isolated group B Streptococcus proteins from culture supernatants that elicit protection in the mouse model (Valtonen, M.V., et al., Microb. Path. 1:191-204 (1986)). They identified, and partially purified, a trypsin resistant group B Streptococcus protein with a molecular weight of 14,000 daltons. Antisera raised to this protein in rabbits protected mice against subsequent challenge with type Ib group B Streptococcus (89% protection). This protein is, by Lancefield's definition, a C protein. However, when antisera raised against this protein were used to immunoprecipitate extracts of group B Streptococcus antigens, a number of higher molecular weight proteins were found to be reactive. This suggested that the 14,000 m.w. protein may represent a common epitope of several group B Streptococcus proteins, or that it is a degradation product found in the supernatants of group B Streptococcus cultures. The diversity in the sizes if C proteins isolated from both the bacterial cells and supernatants suggests that the C proteins may represent a gene family, and maintain antigenic diversity as a mechanism for protection against the immune system.

The range of reported molecular weights and difficulties encountered in purifying individual C proteins are similar to the problems that many investigators have faced in isolating the M protein of group A Streptococcus (Dale, J.B., et al., Infec. and Immun. 46(1):267-269 (1984), Fischetti, V.A., et al., J. Exp. Med. 144:32-53 (1976), Fischetti, V.A. et al ., J. Exp. Med 146:1108-1123 (1977)). The gene for the M protein has now been cloned and sequenced, and found to contain a number of repeated DNA sequences (Hollingshead, S.K., et al., J. Biol. Chem. 261:1677-1686 (1986), Scott, J.R., et al., Proc. Natl. Acad. Sci USA 82:1822-1826 (1986), Scott, J.R., et ala., Infec. and Immun. 52:609-612 (1986)). These repeated sequences may be responsible for post-transcriptional processing that results in a diversity in the size of M proteins that are produced. The mechanism by which this occurs is not understood. The range of molecular weights described for the C proteins of group B Streptococcus might result from a similar process.

Cleat et al. attempted to clone the C proteins by using two preparations of antisera to group B Streptococcus obtained from Bevanger (α and β) to screen a library of group B Streptococcus DNA in E. coli (Bevanger, L. et al., Acta Path. Microbiol. Immunol. Scand. Sect. B. 93:113-119 (1985), Cleat, P.H., et al., Infec. and Immun. 55(5):1151-1155 (1987).). These investigators described two clones that produce proteins that bind to antistreptococcal antibodies. However, they failed to determine whether either of the cloned proteins had the ability to elicit protective antibody, or whether the prevalence of these genes correlated the with group B Streptococcus strains known to carry the C proteins. The role of the cloned gene sequences in the virulence of group B Streptococcus was not investigated. Since the C proteins are defined by their ability to elicit protective antibodies, this work failed to provide evidence that either of the clones encodes a C protein.

### The Conjugated Vaccine of the Present Invention

The present invention surmounts the above-discussed deficiencies of prior vaccines to group B Streptococcus through the development of a conjugate vaccine in which the capsular polysaccharides are covalently linked to a protein backbone. This approach supports the development of a T-cell dependent antibody response to the capsular polysaccharide antigens and circumvents the T-cell independent requirements for antibody production (Baker, C.J., et al., Rev. of Infec. Dis. 7:458-467 (1985), Kasper, D.L. et al., J. Infec. Dis. 153:407-415 (1986).).

In a conjugate vaccine, an antigenic molecule, such as the capsular polysaccharides of group B Streptococcus (discussed above), is covalently linked to a "carrier" protein or polypeptide. The linkage serves to increase the antigenicity of the conjugated molecule. Methods for forming conjugate vaccines from an antigenic molecule and a "carrier" protein or polypeptide are known in the art (Jacob, C.O. et al., Eur. J. Immunol. 16:1057-1062 (1986); Parker, J.M.R. et al., In: Modern Approaches to Vaccines, Chanock, R.M. et al. Eds., pp. 133-138, Cold Spring Harbor Laboratory, Cold Spring Harbor, NY (1983); Zurawski, V.R., et al., J. Immunol. 121:122-129 (1978); Klipstein, F.A. et al., Infect. Immun. 37:550-557 (1982); Bessler, W.G., Immunobiol. 170:239-244 (1985); Posnett, D.N. et al., J. Biol. Chem. 263:1719-1725 (1988); Ghose, A.C. et al., Molec. Immunol. 25:223-230 (1988);).

A prototype model for conjugate vaccines was developed against Hemophilus influenzae (Anderson, P. Infec. and Immun. 39:223-238 (1983); Chu, C. et al., Infect. Immun. 40:245-256 (1983); Lepow, M. Pediat. Infect. Dis. J. 6:804-807 (1987).), and this model may be employed in constructing the novel vaccines of the present invention. Additional methods for producing such a conjugate vaccine are disclosed by Anderson, P.W. et al., European Patent Publication 245,045; Anderson, P.W. et al., U.S. Patent Nos. 4,673,574 and 4,761,283; Frank, R. et al., U.S. Patent No. 4,789,735; European Patent Publication No. 206,852; Gordon, L.K., U.S. Patent No. 4,619,828; and Beachey, E.H., U.S. Patent No. 4,284,537.

The protein backbones for conjugate vaccines such as the Hemophilus influenzae vaccine have utilized proteins that do not share antigenic properties with the target organism from which the bacterial capsular polysaccharides were obtained (Ward, J. et al., In: Vaccines, Plotkin, S.A. et al., Eds., Saunders, Philadelphia, page 300 (1988).

In contrast, the conjugate vaccine of the present invention employs immunogenic proteins of group B Streptococcus as the backbone for a conjugate vaccine. Such an approach is believed to lead to more effective vaccines (Insel, R.A., et al., New Eng. J. Med. (Editorial) 319(18):1219-1220 (1988)). The conjugate, protein-polysaccharide vaccine of the present invention is the first to specifically characterize group B Streptococcus proteins that may be used in a conjugate vaccine. Any protein which is characteristic of group B Streptococcus may be employed as the protein in the conjugate vaccines of the present invention. It is, however, prefered to employ a C protein of a group B Streptococcus for this purpose. As discussed more fully below, plasmids pJMS1 and pJMS23 contain DNA which encode Streptococcus C protein. The most preferred C proteins are those obtained upon the expression of such DNA in bacteria.

As indicated above, the present invention concerns the cloning and expression of genes which encode the protective group B Streptococcus protein antigens. Such proteins are preferably used as the protein backbone to which the capsular polysaccharides of the group B Streptococcus can be conjugated in order to form a conjugate vaccine against these bacteria. In addition, the role of these proteins in the virulence and immunity of group B Streptococcus may be exploited to develop an additional therapy against group B Streptococcus infection. The isolation and characterization of these genes of a bacterial origin allows the manipulation of the gene products to optimize both the adjuvant and antigenic properties of the polypeptide backbone/carrier of the conjugate vaccine.

### Genetic Studies of the C Proteins

The present invention thus concerns the cloning of the C proteins of group B Streptococcus, their role in virulence and immunity, and their ability to serve as an immunogen for a conjugate vaccine against group B Streptococcus.

Despite the extensive literature available on cloning in many groups of Streptococci, only limited genetic manipulations have been accomplished in group B Streptococcus (Macrina, F.L., Ann. Rev. Microbiol. 38:193-219 (1984), Wanger, A.R., et al., Infec. and Immun. 55:1170-1175 (1987)). The most widely used technique in group B Streptococcus has been the development of Tn916 and its use in transposon mutagenesis (Rubens, C.E., et al., Proc. Natl. Acad. Sci. USA 84:7208-7212 (1987), Wanger, A.R., et al., Res. Vet. Sci. 38:202-208 (1985)). However, since it would appear that there is more than one gene for the C proteins and the protective antisera bind to several proteins, screening for the C protein genes by transposon mutagenesis is impractical.

The present invention acomplishes the cloning of the C proteins (and of any other proteins which are involved in the virulence of the group B Streptococcus, or which affect host immunity to the group B Streptococcus) through the use of a novel plasmid vector. For this purpose, it is desirable to employ a cloning vector that could be rapidly screened for expression of proteins which bind to naturally elicited antibodies to group B Streptococcus. Since such antibodies are heterologous polyclonal antibodies and not monoclonal antibodies, it was necessary to that a vector be employed which could be easily screened through many positive clones to identify genes of interest.

A number of techniques were available for screening clones for the expression of antigens that bind to a specific antisera (Aruffo, A., et al., Proc. Natl. Acad. Sci. USA 84:8573-8577 (1987)). The most widely used system, λGT11, was developed by Young and Davis (Huynh, T.V. et al., In: DNA Cloning, A Practical Approach, Vol. 1 (Glover, D.M., Ed.) IRL Press, Washington pp. 49-78 (1985); Wong, W.W., et al., J. Immunol. Methods. 82:303-313 (1985).). This technique allows for the rapid screening of clones expressed in the lysogenic phage whose products are released by phage lysis. Commonly faced problems with this system include the requirement for subcloning DNA fragments into plasmid vectors for detailed endonuclease restriction mapping, preparing probes and DNA sequencing. In addition, the preparation of DNA from phage stocks is cumbersome and limits the number of potentially positive clones that can be studied efficiently. Finally, the preparation of crude protein extracts from cloned genes is problematic in phage vector hosts.

To circumvent these problems, the present invention provides a plasmid vector which was developed for screening cloned bacterial chromosomal DNA for the expression of proteins involved in virulence and/or immunity. The present invention thus further concerns the development and use of an efficient cloning vector that can be rapidly screened for expression of proteins which bind to naturally elicited antibodies to group B Streptococcus. The vector was prepared by modifying the commonly used plasmid cloning vector, pUC12 (Messing, J. et al., Gene 19:269-276 (1982); Norrander, J. et al., Gene 26:101-106 (1983); Vieira, J. et al., Gene 19:259-268 (1982);). The invention concerns the vector described below, and its functional equivalents.

Using this system, plasmid clones can be easily manipulated, mapped with restriction endonucleases and their DNA inserts sequences, probes prepared and gene products studied without the necessity for subcloning. pUC12 is a 2.73 kilobase (kb) high copy number plasmid that carries a ColE1 origin of replication, ampicillin resistance and a polylinker in the lacZ gene (Ausubel, F.M., et al., Current Topics in Molecular Biology; Greene Publ. Assn./ Wiley Interscience, NY (1987)).

Several modifications were made in the polylinker of pUC12 (Aruffo, A., et al., Proc. Natl. Acad. Sci. USA 84:8573-8577 (1987)). The overall plan in altering pUC12 was to modify the polylinker to present identical but non-cohesive BstX1 sites for cloning, to add a "stuffer" fragment to allow for easy separation of the linear host plasmid, and to provide for expression from the lac promoter in all three translational reading frames.

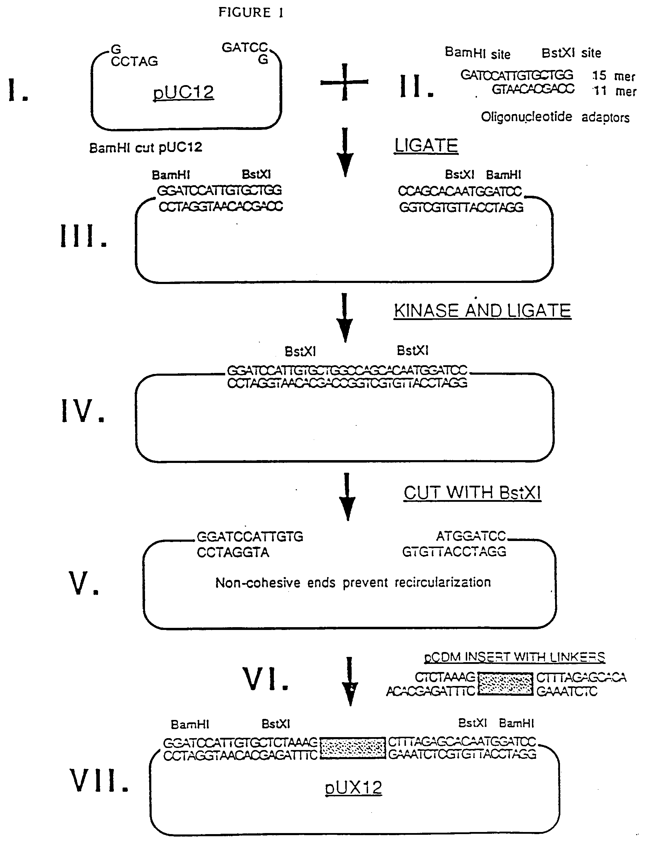

In order to provide a site for the insertion of foreign DNA with a high efficiency and to minimize the possibility for self-ligation of the plasmid, inverted, non-cohesive BstX1 ends were added to the polylinker. As shown in Figure 1, pUC12 was first cut with BamH1 (Step 1) and the plasmid was mixed with two synthetic oligonucleotide adaptors that are partially complementary: a 15-mer (GATCCATTGTGCTGG) and an 11-mer (GTAACACGACC) (Step 2). When the adaptors are ligated into pUC12, two new BstX1 sites are created but the original BamH1 sites are also restored (Step 3). The plasmid was then treated with polynucleotide kinase and ligated to form a closed circular plasmid (Step 4). When this plasmid is treated with BstX1, the resulting ends are identical and not cohesive (both have GTGT overhangs) (Step 5).

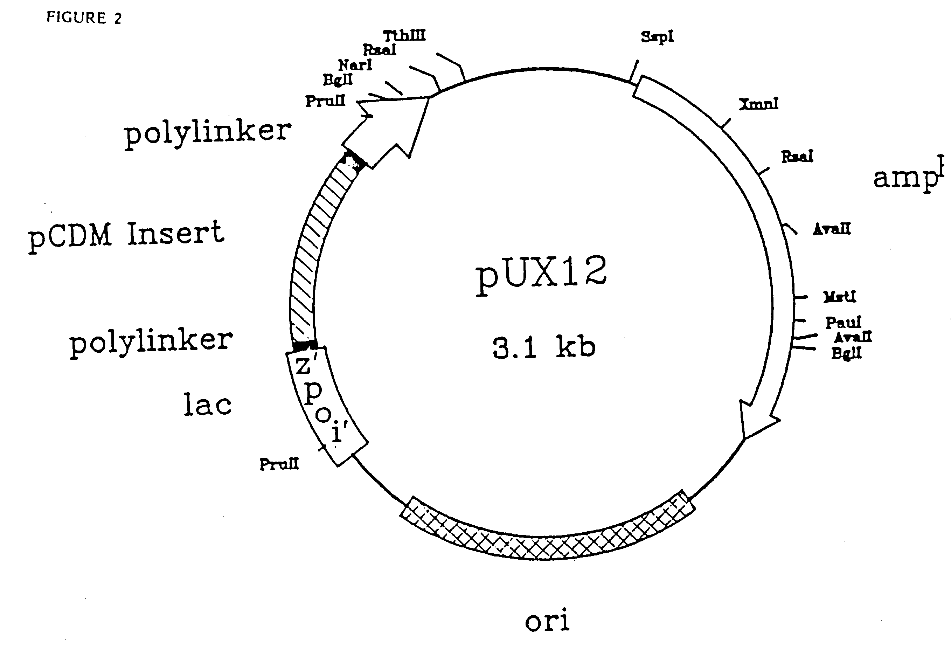

A second modification in the polylinker was done to allow for the purification of the linear plasmid for cloning without contamination from partially cut plasmid that can self-ligate. A blunt end, 365 base pair (bp), FnuD2 fragment was obtained from the plasmid pCDM. This cassette or "stuffer" fragment, which does not contain a BstX1 site, was blunt end ligated to two synthetic oligonucleotides that are partially complementary: a 12-mer (ACACGAGATTTC) and an 8-mer (CTCTAAAG) (Step 6). The resulting fragment with adaptors has 4 bp overhangs (ACAC) that are complementary to the ends of the modified pUC12 plasmid shown in Step 5. The modified pUC12 plasmid was ligated to the pCDM insert with adaptors; the resulting construct, named pUX12, is shown in Figure 2. The pUX12 plasmid can be recreated from plasmids pJMS1 or pJMS23 by excision of the introduced Streptococcus DNA sequences. Alternatively, it may be formed by recombinant methods (or by homologous recombination), using plasmid pUC12.

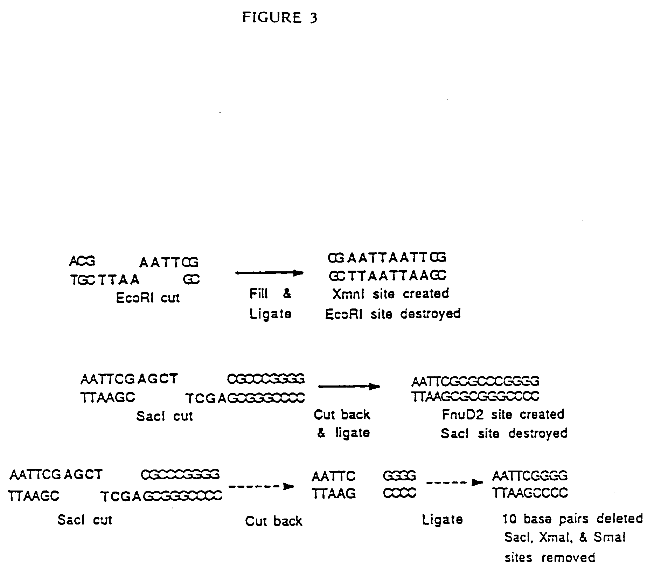

Since PUX12 is to be used as an expression vector, it is preferable to further modified the polylinker such that it will contain all three potential reading frames for the lac promoter. These changes allow for the correct translational reading frame for cloned gene fragments with a frequency of one in six. For example, a cloned fragment can insert in the vector in one of two orientations and one of three reading frames. To construct a +1 reading frame, the pUX12 plasmid was cut with the restriction enzyme EcoR1 which cleaves at a unique site in the polylinker. The single stranded 5' sticky ends were filled in using the 5'-3' polymerase activity of T4 DNA polymerase, and the two blunt ends ligated. This resulted in the loss of the EcoR1 site, and the creation of a new Xmn1 site (Figure 3A). This construction was confirmed by demonstrating the loss of the EcoR1 site and confirming the presence of a new Xmn1 site in the polylinker. In addition, double stranded DNA sequencing on the +1 modified pUX12 plasmid was performed using standard sequencing primers (Ausubel, F.M., et al., Current Topics in Molecular Biology; Greene Publ. Assn./Wiley Interscience, NY (1987)). The DNA sequence showed the addition of 4 base pairs to the polylinker and confirmed the modification of pUX12 to a +1 reading frame. This plasmid is called pUX12+1.

In order to construct a -1 reading frame, the pUX12 vector was cut with the restriction enzyme SacI which cuts at a unique site in the polylinker of pUX12. The single stranded 3' sticky ends were cut back to blunt ends using the 3'-5' exonuclease activity of T4 polymerase, and the resulting blunt ends ligated. The resulting sequence should eliminate the Sac1 site while resulting in a new FnuD2 site (Figure 3B). However, restriction mapping of the pUX12-1 plasmids showed that while the Sac1 site was absent, there was no FnuD2 site present. In addition, the Sma1/Xma1 sites on the polylinker were no longer present. Several potential pUX12-1 constructs were sequenced from mini-prep, double-stranded DNA. Of the six modified plasmids sequenced, one was found with ten nucleotides absent, thereby creating a -1 reading frame (Figure 3C). This suggests that the T4 DNA polymerase has additional exonuclease activity and cuts back additional double stranded portions of the polylinker. Nevertheless, the resulting plasmid had a -1 reading frame. The plasmid was named pUX12-1.

The use of the pUX-12 vectors in the cloning of antigenic proteins of group B Streptococcus are discussed in detail in the Examples below. In brief, DNA derived from group B Streptococcus, or complementary to such DNA is introduced into the pUX12, pUX12+1 or pUX12-1 vectors and transformed into Escherichia coli. The cloned DNA is expressed in E. coli and the cellular lysate is tested to determine whether it contains any protein capable of binding to antisera to group B Streptococcus.

There are a number of potentially interesting modifications of pUX12 that could increase its utility. For example, the lac promoter could be replaced by another promoter, the origin of replication could be modified to produce a lower copy number vector and the drug resistance marker could be changed.

The present invention concerns a vaccine comprising a polysaccharide (such as the capsular polysaccharide) which is characteristic of the group B Streptococcus conjugated to a protein which is also characteristic of the group B Streptococcus. The "polysaccharide" and "protein" of such a conjugated vaccine may be identical to a molecule which is characteristic of the group B Streptococcus, or they may be functional derivatives of such molecules. Examples of functional derivatives include fragments of a natural protein, and/or variants of a natural protein (such as proteins having changes in amino acid sequence but which retain substantially the same immunogenic, virulence or antigenic properties as are exhibited by the natural molecule). For the purposes of the present invention, any polysaccharide which when introduced into a mammal (either animal or human) elicits antibodies which are capable of reacting with group B Streptococcus may be employed. Examples of the preferred polysaccharides of the present invention include the capsular polysaccharide of the group B Streptococcus, or their equivalents. For the purposes of the present invention, any protein which when introduced into a mammal (either animal or human) either elicits antibodies which are capable of reacting a protein expressed by group B Streptococcus, or which increases the immunogenicity of a polysaccharide to elicit antibodies to a polysaccharide of the group B Streptococcus may be employed. Examples of the preferred proteins of the present invention include the C proteins of the group B Streptococcus, or their equivalents.

As used herein, a polysaccharide or protein is "characteristic" of a bacteria if it substantially similar in structure or sequence to a molecule naturally asociated with the bacteria. The term is intended to include both molecules which are specific to the organism, as well as molecules which, though present on other organisms, are involved in the virulence or antigenicity of the bacteria in a human or animal host.

The vaccine of the present invention may confer resistance to group B Streptococcus by either passive immunization or active immunization. In one embodiment of passive immunization, the vaccine is provided to a host (i.e. a human or mammal) volunteer, and the elicited antisera is recovered and directly provided to a recipient suspected of having an infection caused by a group B Streptococcus.

The ability to label antibodies, or fragments of antibodies, with toxin labels provides an additional method for treating group B Streptococcus infections when this type of passive immunization is conducted. In this embodiment, antibodies, or fragments of antibodies which are capable of recognizing the group B Streptococcus antigens are labeled with toxin molecules prior to their administration to the patient. When such a toxin derivatized molecule binds to a group B Streptococcus cell, the toxin moiety will cause the death of the cell.

In a second embodiment, the vaccine is provided to a female (at or prior to pregnancy or parturition), under conditions of time and amount sufficient to cause the production of antisera which serve to protect both the female and the fetus or newborn (via passive incorporation of the antibodies across the placenta).

The present invention thus concerns and provides a means for preventing or attenuating infection by group B Streptococcus, or by organisms which have antigens that can be recognized and bound by antisera to the polysaccharide and/or protein of the conjugated vaccine. As used herein, a vaccine is said to prevent or attenuate a disease if its administration to an individual results either in the total or partial attenuation (i.e. suppression) of a symptom or condition of the disease, or in the total or partial immunity of the individual to the disease.

The administration of the vaccine (or the antisera which it elicits) may be for either a "prophylactic" or "therapeutic" purpose. When provided prophylactically, the compound(s) are provided in advance of any symptom of group B Streptococcus infection. The prophylactic administration of the compound(s) serves to prevent or attenuate any subsequent infection. When provided therapeutically, the compound(s) is provided upon the detection of a symptom of actual infection. The therapeutic administration of the compound(s) serves to attenuate any actual infection.

The anti-inflammatory agents of the present invention may, thus, be provided either prior to the onset of infection (so as to prevent or attenuate an anticipated infection) or after the initiation of an actual infection.

A composition is said to be "pharmacologically acceptable" if its administration can be tolerated by a recipient patient. Such an agent is said to be administered in a "therapeutically effective amount" if the amount administered is physiologically significant. An agent is physiologically significant if its presence results in a detectable change in the physiology of a recipient patient.

As would be understood by one of ordinary skill in the art, when the vaccine of the present invention is provided to an individual, it may be in a composition which may contain salts, buffers, adjuvants, or other substances which are desirable for improving the efficacy of the composition. Adjuvants are substances that can be used to specifically augment a specific immune response. Normally, the adjuvant and the composition are mixed prior to presentation to the immune system, or presented separately, but into the same site of the animal being immunized. Adjuvants can be loosely divided into several groups based upon their composition. These groups include oil adjuvants (for example, Freund's complete and incomplete), mineral salts (for example, AlK(SO₄)₂, AlNa(SO₄)₂, AlNH₄(SO₄), silica, kaolin, and carbon), polynucleotides (for example, poly IC and poly AU acids), and certain natural substances (for example, wax D from Mycobacterium tuberculosis, as well as substances found in Corynebacterium parvum, or Bordetella pertussis, and members of the genus Brucella. Among those substances particularly useful as adjuvants are the saponins such as, for example, Quil A. (Superfos A/S, Denmark). Examples of materials suitable for use in vaccine compositions are provided in Remington's Pharmaceutical Sciences (Osol, A., Ed., Mack Publishing Co., Easton, PA, pp. 1324-1341 (1980).).

The therapeutic compositions of the present invention can be administered parenterally by injection, rapid infusion, nasopharyngeal absorption (intranasopharangeally), dermoabsorption, or orally. The compositions may alternatively be administered intramuscularly, or intravenously. Compositions for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Carriers or occlusive dressings can be used to increase skin permeability and enhance antigen absorption. Liquid dosage forms for oral administration may generally comprise a liposome solution containing the liquid dosage form. Suitable forms for suspending liposomes include emulsions, suspensions, solutions, syrups, and elixirs containing inert diluents commonly used in the art, such as purified water. Besides the inert diluents, such compositions can also include adjuvants, wetting agents, emulsifying and suspending agents, or sweetening, flavoring, or perfuming agents.

Many different techniques exist for the timing of the immunizations when a multiple administration regimen is utilized. It is possible to use the compositions of the invention more than once to increase the levels and diversities of expression of the immunoglobulin repertoire expressed by the immunized animal. Typically, if multiple immunizations are given, they will be given one to two months apart.

According to the present invention, an "effective amount" of a therapeutic composition is one which is sufficient to achieve a desired biological effect. Generally, the dosage needed to provide an effective amount of the composition will vary depending upon such factors as the animal's or human's age, condition, sex, and extent of disease, if any, and other variables which can be adjusted by one of ordinary skill in the art.

The antigenic preparations of the invention can be administered by either single or multiple dosages of an effective amount. Effective amounts of the compositions of the invention can vary from 0.01-1,000 µg/ml per dose, more preferably 0.1-500 µg/ml per dose, and most preferably 10-300 µg/ml per dose.

Having now generally described the invention, the same will be more readily understood through reference to the following examples which are provided by way of illustration, and are not intended to be limiting of the present invention, unless specified.

### EXAMPLE 1

### CLONING EFFICIENCY OF THE pUX12 VECTORS

Several experiments were designed to test the cloning efficiency of the pUX12 vectors and to determine whether the modified reading frames transcribed correctly. The results of these experiments will be briefly summarized below:
1. To clone a DNA fragment into pUX12, the three constructs, pUX12 (the original "zero" reading frame construction), pUX12+1 and pUX12-1, were mixed in equimolar concentrations. The plasmids were then cut with BstX1 to cleave the stuffer fragment within the polylinker. The stuffer fragment was separated from the plasmid using either low melting point agarose or a potassium acetate gradient (Aruffo, A., et al., Proc. Natl. Acad. Sci. USA 84:8573-8577 (1987), Ausubel, F.M., et al., Current Topics in Molecular Biology; Greene Publ. Assn./ Wiley Interscience, NY (1987)). The DNA to be cloned was cut with a restriction enzyme that gives blunt ends (any such restriction enzyme may be employed). If necessary, double stranded DNA with signal stranded ends can be modified to create blunt ends. The blunt ends of the DNA fragments were mixed with the two synthetic oligonucleotide adaptors. These are the same 12-mer and 8-mer used in preparing the stuffer fragment. The modified DNA fragments were separated from the unincorporated synthetic oligonucleotides on a potassium acetate gradient. These fragments were then ligated into the linear pUX12 family of plasmids and used to transform E. coli.
   To verify that the pUX12 vectors self-ligate at a low frequency under conditions optimize for the cloning of inserts with adaptors, a second drug resistance marker was cloned into pUX12. pUX12 has a β-lactamase gene and carriers resistance to ampicillin (amp^{R}). The rationale for cloning a second marker was to compare the ratio of clones that contained both drug resistance markers to those pUX12 plasmids that self-ligated under typical cloning conditions and therefore only expressed resistance to ampicillin. The tetracycline resistance gene (tet^{R}) from the plasmid pBR322 was cloned into the polylinker of pUX12 with the adaptors described above. A group of test ligations were run to establish the optimal concentration of oligonucleotide adaptor to fragment ends, and the ratio of modified insert to linear pUX12 plasmid for ligation and transformation. By using the tet^{R} gene as a marker, we were able to determine cloning parameters so that greater than 99% of the transformants selected on ampicillin containing plates also carried the tet^{R} marker. Thus, the frequency of self-ligation is very low in this system and it is not necessary to screen for the presence of an insert in the polylinker prior to screening a library in pUX12.
2. To confirm the position of the translational reading frame in the polylinker of pUX12, a structural gene whose sequence and product are known, and that lacks its own promoter, was cloned. For this purpose, a mutant of the tox structural gene carried on the plasmid (Costa, J.J., et al., J. Bacteriol. 148(1):124-130 (1981), Michel, J.L., et al., J. Virol. 42:510-518 (1982) was chosen. The plasmid, pABC402, was treated simultaneously with the restriction endonucleases ApAI and HindIII (Bishai, W.R., et al., J. Bacteriol. 169:1554-1563 (1987), Bishai, W.R., et al., J. Bacteriol. 169:5140-5151 (1987). The ApaI site is within the structural gene near the N-terminal and the HindIII site lies just outside of the C-terminal of the tox gene. This 1.2 kb restriction fragment was separated from the remaining 4.1 kb of the pABC402 vector using low melting point agarose.
   To create blunt ends for cloning, the tox fragment was treated with T4 DNA polymerase. The exonuclease activity of the polymerase cut back the ApaI 3' ends and the polymerase activity filled in the 5' overhand at the HindIII site (Maniatis, T. et al., Molecular Cloning, A Laboratory Manual, Cold Spring Harbor Press, Cold Spring Harbor, NY (1982)). This purified fragment with blunt ends was ligated into the mixture of pUX12 that contains all three reading frames. Individual transformants were randomly picked and screened by restriction mapping to determine the orientation and reading frame of the inserts. In addition, the nucleotide sequences of the polylinker/adaptor/insert regions were determined. All six potential orientation and reading frame combinations were identified. Finally, extracts from these clones were screened using Western blots probed with antisera to diphtheria toxin (Blake, M.S., et al., Analyt. Biochem. 136:175-179 (1984), Murphy, J.R. et al., Curr. Topics Microbiol. and Immun. 118:235-251 (1985)).

Reactive toxin related proteins were only detected from clones that contained the structural gene in the correct orientation and reading frame. This plasmid is called pUDTAH-1; the DNA sequence of the polylinker and beginning of the tox structural gene is shown in Table 1. The depisted sequence is the DNA sequence of the beginning of the tox' structural gene in pUDTAH-1. ATG is the start signal for the transcript (lacZ'), GAT begins the modified polylinker of pUX12 and GCC starts the correct translational reading frame for the tox' gene.

**TABLE 1**

| SEQUENCES OF PLASMID pUDTAH | | |
|---|---|---|
| ATGACCATGATTACGAATTCGAGCTCGCCCGGG | **GATCCATTGTGCTGGAAAG** | CCACC |
| POLYLINKER (ATG=LacZ Translation Initiation Codon) | OLIGONUCLEOTIDE ADAPTORS | DIPHTHERIA TOX' GENE |

### EXAMPLE 2

### PURIFICATION OF CHROMOSOMAL DNA FROM GROUP B STREPTOCOCCUS

To accomplish the purification of chromosomal DNA from group B Streptococcus chromosomal DNA was isolated from the A909 strain of group B Streptococcus (Lancefield, R.C., et al., J. Exp. Med. 142:165-179 (1975)) by the method of Hull et al. (Hull, R.A., et al., Infect. and Immun. 33:933-938 (1981)) as modified by Rubens et al. (Rubens, C.E., et al., Proc. Natl. Acad. Sci USA 84:7208-7212 (1987)). In brief, mutanolysin was used to convert the group B Streptococcus strain A909 (Ia/c) strain into protoplasts. The resulting surface extract was found to contain numerous proteins that immunoreact with protective antisera raised to the intact bacteria. An insoluble protein fraction was partially purified using conventional column chromatography. Two fractions, including one which was highly concentrated for a single 14 kilodalton (kd) species, were used to immunize rabbits. Antisera raised against these partially purified group B Streptococcus proteins were found to be able to confer passive protection in a mouse virulence assay against a heterologous capsule type of group B Streptococcus which carries the C proteins.

Group B Streptococcus DNA was purified by centrifugation in a buoyant-density cesium chloride (CsCl) gradient, and the chromosomal DNA was dialyzed exhaustively against TAE buffer, pH 8.0 (Maniatis, T. et al., Molecular Cloning, A Laboratory Manual, Cold Spring Harbor Press, Cold Spring Harbor, NY (1982). The A909 strain of group B Streptococcus has a type 1 capsule, expresses the C proteins and has been used previously in studies of the C proteins (Valtonen, M.V., et al., Microb. Path. 1:191-204 (1986)). It is also the strain of group B Streptococcus that was used in preparing the protective antisera for screening.

The yield of Group B Streptococcus chromosomal DNA averages 3 to 5 mg for each 500 ml of an overnight culture of group B Streptococcus. The purified DNA was digested separately with 24 commonly used restriction endonucleases and the resulting fragments were run on a 1.0% agarose gel. A wide range of enzymes were chosen, including those that have unique sites on the polylinkers commonly used in cloning vectors. Ethidium bromide (EtBr) staining of the gel showed that all of the restriction enzymes yielded a distribution of discrete fragment sizes of group B Streptococcus DNA. This suggests that group B Streptococcus DNA is not modified for any of the restriction enzymes tested.

In order to determine whether there were any inhibitors present to block ligation of the DNA, the restriction endonuclease digestions described above were ethanol precipitated, placed in a ligation buffer and incubated overnight at 14°C with DNA ligase. These samples were again run on a 1.0% agarose gel and stained with EtBr. The resulting restriction patterns showed a higher molecular weight distribution. Therefore, there was no inhibition of the ligation of group B Streptococcus DNA.

### EXAMPLE 3

### PREPARATION OF A LIBRARY OF GROUP B STREPTOCOCCUS CHROMOSOMAL DNA

The preparation of a library of group B Streptococcus chromosomal DNA in pUX12 and its transformation into E. coli was performed as follows. To cleave the group B Streptococcus chromosomal DNA for cloning, four restriction enzymes were chosen that give a broad distribution of restriction fragment sizes The pUX12 vector and adaptors are most efficient when blunt ended fragments are cloned. The enzymes chosen recognize four base pair sites and leave blunt ends. Group B Streptococcus DNA was partially digested individually with Alu1, FunD2, HaeIII and RsaI.

The resulting fragments were mixed, purified with phenol/chloroform, ethanol precipitated and resuspended in a ligation buffer (Maniatis, T. et al., Molecular Cloning, A Laboratory Manual, Cold Spring Harbor Press, Cold Spring Harbor, NY (1982)). One µg of the group B Streptococcus DNA fragments was mixed with 3 µg of the 12-mer and 2 µg of the 8-mer oligonucleotide adaptors. Three microliters of T4 DNA ligase (600 units, New England Biolabs), were added and the reaction was maintained overnight at 14°C. The free linkers were separated from the group B Streptococcus DNA fragments on a potassium acetate velocity gradient (Aruffo, A., et al., Proc. Natl. Acad. Sci. USA 84:8573-8577 (1987)).

The pUX12 plasmid containing all three translational reading frames was digested with BstX1 and the stuffer fragment was removed using a low melting point agarose gel. The group B Streptococcus library was prepared by mixing 10 ng of the adapted group B Streptococcus fragments with 100 ng of the linear pUX12 vector in 100 µl of ligation buffer to which 0.1% T4 DNA ligase was added. The ligation reaction was maintained overnight at 14°C and then used to transform the MC1061 strain of E. coli on plates containing ampicillin (Ausubel, F.M., et al., Current Topics in Molecular Biology (1987)).

Sixteen of the resulting transformants were isolated, grown overnight in LB and plasmid DNA isolated by mini-preps. The plasmid DNA was digested with BamH1, and run on a 1.0% agarose gel. All of the plasmids screened contained inserts in the pUX12 vector, and the average insert size was 1.4 kb. To date, the plasmid DNA obtained from over 200 clones have been screened and only one clone was found that appeared to lack an insert in the polylinker.

### EXAMPLE 4

### CHARACTERIZATION OF PROTECTIVE ANTISERA TO BE USED IN SCREENING THE LIBRARY

As discussed earlier, the C proteins have been partially purified by a variety of techniques and protective antisera have been partially purified by a variety of techniques and protective antisera have been prepared by a number of investigators (Bevanger, L. et al., Acta Path. Microbio. Scand. Sect. B. 93:113-119 (1985), Russell-Jones, G.J., et al., J. Exp. Med. 160:1476-1484 (1984), Wilkinson, H.W., et al., Infec. and Immun. 4:596-604 (1971)).

A set of experiments was performed to duplicate the work of Valtonen, Kasper and Levy who isolated a 14,000 mw protein from supernatants of group B Streptococcus that elicits protective antibody (Valatonen, M.V., et al., Microb. Path. 1:191-204 (1982)). This experiment revealed that when proteolytic inhibitors to the supernatants of group B Streptococcus cultures are added prior to the concentration and purification of the C proteins (Wong, W.W., et al., J. Immunol. Methods. 82:303-313 (1985)), the 14,000 mw protein was no longer a prominent protein in the supernatant. This indicated that this protein results from the proteolysis of larger molecular weight C proteins in the supernatants of group B Streptococcus cultures.

### EXAMPLE 5

### OPTIMIZING CONDITIONS FOR SCREENING FOR EXPRESSION IN A PLASMID-BASED VECTOR

As discussed above, the most commonly used vectors for the detection of expression are based on λGT11 (Young, R.A., et al., Proc. Natl. Acad. Sci USA 80:1194-1198 (1983)). We were able to increase the sensitivity of detection of expression from the pUX12 plasmid vector by combining two previously described procedures for antibody screening of bacterial colonies. The transformants from the library were plated overnight and the resulting colonies transferred to nitrocellulose filters (Bio-Rad). The colonies were lysed by placing the filters in an atmosphere saturated with chloroform (CHCL₃) in a closed container for 30 minutes. The filters were then placed in a lysis₃ buffer and incubated overnight as described by Helfman et al. (Helfman, D.M., et al., Proc. Natl. Acad. USA 80:31-35 (1983)). The antibody screening was done utilizing commercially prepared E. coli lysate (ratio 1:200) and Horseradish Peroxidase Conjugated, Affinity Purified Goat Anti-Rabbit IgG (ratio 1:3000) in the Express-Blot Assay Kit prepared by Bio-Rad Laboratories. By pretreating the colonies with chloroform and the overnight incubation with DNase and lysozyme described above, it was possible to reduce the ratio of primary antibody required from 1:500 to 1:5000.

### EXAMPLE 6

### INITIAL ANALYSIS OF POSITIVE CLONES AND THEIR PROTEIN PRODUCTS

The library of group B Streptococcus chromosomal DNA in the pUX12 vector was screened with the above-discussed protective anti-C proteins antisera. The group B Streptococcus library had an average fragment size of 1.4 kb. Transformants were screened as described above, and then subcloned and rescreened with the antisera three times. Of 20,000 clones screened, there were 35 independently isolated clones that reacted with the protective antisera. The clones were denominated S1-S35, and the plasmids containing the clones were denominated pJMS1-pJMS35. The clones ranged in size from 0.9 to 13.7 kb and have an average size of 4.5 kb.

Plasmid DNA was isolated from the clones by minipreps and the inserts surveyed with four restriction endonucleases. Fourteen of the clones can be divided into three groups based on sharing identical insert sizes and common restriction endonuclease mapping patterns within each group. Clones S1 and S23, discussed below, were found to be members of different groups.

By further comparing the restriction patterns of the individual clones it was possible to identify 24 clones that shared common restriction fragments. Clones S1 and S23 were not found to share any common restriction fragments.

Extracts of the clones were prepared, run on Western blots and probed with the antisera used in screening the library. Six size classes of protein antigens were identified (A-F). By combining data from the restriction endonuclease mapping and the Western blots it was possible to classify 24 of the 35 clones into 6 different protein antigen patterns (Table 2). This initial classification was done only to get a rough survey of the potential number of genes involved. S1 was found to be 3.5 kd in size, and to belong to antigen protein pattern A. S23 was found to be 13.7 kd in size, and to belong to antigen protein pattern D.

**TABLE 2**

| PRELIMINARY CLASSIFICATION OF THE GROUP B STREPTOCOCCUS C PROTEIN CLONES | | | | |
|---|---|---|---|---|
| Protein Profile | Number of clones | Molecular Weight of insert (kb) | Coding Capacity of DNA insert | Size of Antigen (in daltons) |
| A | 6 | 3.5 | 136,000 | 115,000 |
| B | 3 | 1.9 | 76,000 | 50,000 |
| C | 7 | 4.4 | 174,000 | 130,000 |
| D | 6 | 13.7 | >500,000 | 110,000 |
| E | 1 | 1.7 | 67,000 | 50,000 |
| F | 1 | 0.9 | 36,000 | 15,000 |

When Western blots of extracts of the clones were probed with antisera to a group B Streptococcus strain that does not express the C proteins, only one group of clones was positive (Protein Profile B). This indicates that the majority of positive clones express proteins that are unique to strains that carry the C proteins; these proteins are not common to all strains of group B Streptococcus.

### EXAMPLE 7

### CHARACTERIZATION OF THE CLONED GENE SEQUENCES

The actual number of C proteins that are expressed by group B Streptococcus has not been determined. Recent immunological studies by Brady et al. characterizing C protein typing antisera from the C.D.C. identified four separate antigens (Brady, L.J. et al., J. Infect. Dis. 158(5):965-972 (1988)). Preliminary genetic and immunological characterization of the putative C protein clones of group B Streptococcus suggests that four or five genes encode proteins that are present on strains of group B Streptococcus that are known to carry the C proteins. Two groups of experiments were conducted to determine whether the cloned gene products represent C proteins.

As discussed above, studies of the C proteins had defined two phenotypes: one group of proteins that was sensitive to degradation by pepsin but not trypsin (called TR or α) and another group of proteins that was sensitive to degradation by both pepsin and trypsin (called TS or β) (Johnson, D.R., et al., J. Clin. Microbiol. 19:506-510 (1984) , Russell-Jones, G.J., et al., J. Exp. Med. 160:1476-1484 (1984)).

The typing antisera, a and β, were used to screen the cloned gene products on Western blots (Bevanger, L., et al., Acta Path. Microbio. Scand. Sect. B. 87:51-54 (1979); Bevanger, L., et al., Acta. Path. Microbio. Scand. Sect. B. 89:205-209 (1981); Bevanger, L. et al., Acta. Path. Microbio. Scand. Sec. B. 91:231-234 (1983); Bevanger, L. et al., Acta. Path. Microbio. Scand. Sect. B. 93:113-119 (1985); Bevanger, L., et al., Acta. Path. Microbiol. Immuol. Scand. Sec. B. 93:121-124 (1985)).

The α typing sera identified Protein Profile D, and the β typing antisera identified Protein Profile A. These proteins were subjected to digestion with pepsin and trypsin. Protein Profile D is sensitive to pepsin but not trypsin, and Protein Profile A is sensitive to both pepsin and trypsin. These results are consistent with previous studies and confirm that at least two of the C protein genes have been cloned.

The most important and characteristic property of the C proteins is their ability to elicit protective antibodies against group B Streptococcus strains that express C proteins. Several approaches could be used to prepare antisera against the cloned gene products. For example, lysates of the E. coli clones could be directly injected into rabbits in order to determine if the lysates contain proteins capable of eliciting antibodies to any of the E. coli or group B Streptococcus proteins introduced. The resulting antisera can be preabsorbed with a lysate of E. coli prior to testing the antisera to reduce the number of cross-reacting antibodies. Such a lysate can be used to reduce the number of cross-reacting antibodies in both colony blots used for screening the clones for expression and in Western blots used to study both cellular extracts of group B Streptococcus and partially purified group B Streptococcus proteins.

Representative clones from Protein Profiles A, B and D are sonicated and injected into rabbits to raise antisera against the cloned group B Streptococcus protein antigens (Lancefield, R.C., et al., J. Exp. Med. 142:165-179 (1975), Valtonen, M.V., et al., Microb. Path. 1:191-204 (1986)). The control rabbits are injected with E. coli that carries pUX12 without an insert in the polylinker. The antisera is preadsorbed with an E. coli lysate and screened first on Western blots against extracts of the clones in the library. Therefore, it is possible to determine if there are cross-reacting epitopes between the clones and to confirm that these antisera are directed against the cloned proteins identified during the preliminary round of screening.

Alternatively, the preadsorbed antisera may be tested in the mouse protection model. In this classic model, the mice are injected intraperitoneally with rabbit antisera (Lancefield, R.C., et al., J. Exp. Med. 142:165-179 (1975)). The following day they are again injected intraperitoneally with an LD₉₀ of viable group B Streptococcus that are known to carry C proteins. The endpoint is the death of the mice over a 48 hour period.

In order to test the immunogenicity of the proteins expressed by the cloned gene sequences, Escherichia coli cells containing pJMS1 and pJMS23 were grown, and used to prepare cellular extracts. These extracts were then used to immunize rabbits. Antisera raised in response to immunization with the S1 and the S23 extracts were tested using the mouse protection model.

When the mouse protection model experiment was performed, the antisera raised from the clones representing Protein Profiles A and D (S1 and S23, respectively), were each found to be protective. Antisera from a clone representing Protein Profile C was not protective and the control antisera also did not show protection. The antisera raised against the clones expressing Protein Profile C also binds to proteins extracted from strains of group B Streptococcus that do not carry the C protein. Therefore, this group of clones do not encode C proteins. In summary, five of the six groups of clones do not encode proteins that are unique to strains of group B Streptococcus that express C proteins.

The initial biochemical, immunological and functional analysis of two of the groups of clones demonstrates that at least two C proteins genes (S1 and S23) have been successfully cloned. This is the first demonstration that single polypeptide gene products cloned from group B Streptococcus can elicit protective immunity. Antibodies to S1 was found to be able to bind two bands of the A909 extract at 50 and 60 kd. Antibodies to S23 were found to be able to bind to a regularly repeating pattern of bands in the group B Streptococcus surface extract which ranged in MW from >180 kd to 40 kd. A monoclonal antibody derived from the A909 extract showed this same repeating pattern of immunoreactivity. This indicates that a single epitope was recognized in different molecular weight proteins and suggests a regularly repeating structure. The proteins recognized by the S1 anti serum were susceptible to pepsin and trypsin degradation whereas those recognized by the S23 antiserum were susceptible to pepsin but not to trypsin. This experiment shows that these proteins partially purified from group B Streptococcus and expressed from the group B Streptococcus cloned genes represent the alpha and beta antigens of the C protein of group B Streptococcus.

The 35 potential C protein clones described above may be evaluated both genetically and immunologically to determine the number of genes that are present. In addition, the isolation of these clones permits the genes which confer protective immunity to group B Streptococcus infection may be identified. It is likely that the protective antisera used to obtain the initial clones also detected proteins other than the C proteins. The use of such other proteins in a therapy against Streptococcus B infection is also contemplated by the present invention. Since a major goal of the present invention is the isolation and identification of the proteins involved in immunity, antisera prepared against the proteins expressed by these clones may be studied in the mouse protection model. Those genes that express proteins that are protective are preferred proteins for a conjugate vaccine.

As discussed above, the initial screening of group B Streptococcus chromosomal DNA in an E. coli/pUX12 vector library with protective antisera resulted in 35 independently isolated clones. By combining data from restriction endonuclease mapping of the cloned fragments and Western blots of protein extracts from the clones, it was possible to tentatively classify 24 of the 35 clones into 6 different protein antigen patterns (Table 2). This survey permitted a determination of the potential number of genes isolated.

To further characterize such clones, colony blots are preferably used to determine which clones share common DNA sequences. For such blots, a single colony of each of the clones is placed in a well of microtiter dish containing LB broth and grown at 37°C overnight. Control colonies include the host E. coli strain and the E. coli strain containing pUX12. The overnight cultures are transferred onto a nitrocellulose filter on an agar plate containing the same culture medium. These plates are grown up over 8 hours at 37°C and the nitrocellulose filter containing the freshly grown colonies is prepared to be screened for DNA-DNA hybridization. The probes are prepared from the group B Streptococcus DNA inserts in the pUX12 library. Mini-preps are used to obtain plasmid DNA from the clones. The polylinker in pUX12 has both a BamH1 and BstX1 site on either side of the insert; therefore, the group B Streptococcus insert is excised from the plasmid using either BamH1 or BstH1. Fortunately, the chromosomal DNA of group B Streptococcus contains few BamH1 sites and many of the inserts are removed from the vector in one fragment as the result of digestion with BamH1. Low melting point agarose is used to separate the plasmid vector from the inserts. The inserts will be cut from the agarose gel and directly label led by random prime labelling. The labelled inserts are then used to probe the colony blots. This results in the identification of clones that share DNA sequences.

Thus, on the basis of the information obtained from the colony blots described above, the 35 clones are placed into groups that share DNA sequences. These groups are mapped with multiple restriction endonucleases to determine the relationship of each clone to the others within that region of the DNA. Since the host plasmid, pUX12, contains many unique restriction endonucleases sites that are present only in the polylinker, much of the restriction mapping can be done utilizing the plasmid mini-prep DNA without needing to purify the inserts separately. By combining the colony blot data with detailed restriction mapping it is possible to get a reasonable assessment of the number of genetic loci involved. If some of the groups of clones do not represent the genes of interest in their entirety, it may be necessary to use these clones to isolate other more complete copies of the genes from the chromosomal library. However, given the large average size distribution of the initial 35 clones isolated, it is likely that some may represent a complete open reading frame.

Before proceeding with a genetic analysis, antisera is preferably prepared against the cloned gene products, and utilized in the mouse protection model to determine the ability of these antisera to protect against infection with group B Streptococcus (Lancefield, R.C., et al., J. Exp. Med. 142:165-179 (1975), Valtonen, M.V., et al. Microb. Path. 1:191-204 (1986)).

A clone whose expressed protein is able to elicit protective antibodies is a preferred candidate for use in a conjugate vaccine. Clones whose expressed protein is fails to elicit protective antibodies may be further analyzed to determine whether they are also candidates for a vaccine. Since the C proteins are membrane assocated, a failure of protein expressed by a clone to elicit protective antibodies may reflect the fact that the protein may not be stable in E. coli, and in a high copy number vector. This problem has occurred in cloning other membrane proteins from both group A and group B Streptococcus (Kehoe, M. et al., Kehoe, M., et al., Infect. and Immun. 43:804-810 (1984), Schneewind, O., et al., Infec. and Immun. 56:2174-2179 (1988)). Several of the 35 clones isolated in the preliminary studies show a small colony morphology. In addition, some of these clones are unstable and have been found to delete part of the group B Streptococcus DNA insert from the pUX12 polylinker. There are several techniques that can be used to stabilize these clones including: cloning into a low copy number vector or behind a promoter that can be down-regulated, growing the clones at 30°C instead of 37°C, cloning into a vector that has been adapted to accumulate membrane proteins. In addition, it is possible to transform the plasmids into an E. coli host, pcnB, that restricts the copy number of pBR322 derived plasmids like pUX12 (Lopilato J., et al., Mol. Gen. Genet. 205:285-290 (1986)).

A failure of a clone to express protein which elicits protective antibodies may also indicate that the expressed protein lacks an epitope which is important for protection. This could be the case if the entire gene was not cloned or could not be expressed in E. coli. It might also be problem if there is post-transcriptional processing of the C proteins in group B Streptococcus but not for the cloned C protein genes in E coli. It might be necessary either to subclone out the complete gene and/or transfer it into an alternate host background where it can be expressed.

A failure of a clone to express protein which elicits protective antibodies may also indicate that antibodies elicited from antigens produced in Escherichia coli may differ from those elicited from an animal by the native C proteins on group B Streptococcus. In addition, the lysed bacterial extracts used to immunize the rabbits contain a number of E. coli protein antigens. Therefore, it may be necessary to obtain antisera for testing in the animal model from partially purified gene products instead of from the entire organism.

Any cloned group B Streptococcus proteins that are able to elicit protective antibodies can be called C proteins. The antisera prepared for this group of experiments will also be used for localizing these protein.

### EXAMPLE 8

### MAPPING, CHARACTERIZATION AND SEQUENCING OF THE C PROTEIN GENES

In order to further characterize the C protein genes, a fine structure genetic map of C protein gene clones described above may be prepared and their DNA sequence(s) determined. Such mapping is preferably accomplished utilizing genomic Southern blots. By determining the DNA sequences of the C protein genes, one can determine the structure of the genes including their ribosomal binding sites, potential promoters, signal sequences, and any unusual repetitive sequences. The DNA sequences are preferably compared to a library of known DNA sequences to see if there is homology with other genes that have been characterized. In addition, the protein sequences of the C proteins can be determined from DNA sequences of their genes. It is often possible to make predictions about the structure, function and cellular location of a protein from the analysis of its protein sequence.

Genomic Southern blots are, thus, preferably used to determine if any of the genes are linked. For this technique, group B Streptococcus chromosomal DNA is digested individually with several different restriction endonucleases that identify sequences containing six or more base pairs. The purpose is to obtain larger segments of chromosomal DNA that may carry more than one gene. The individual endonuclease digestions are then run out on an agarose gel and transferred onto nitrocellulose. The Southern blots are then probed with the labelled inserts derived from the above-described library. If two clones that did not appear related by the colony blots or endonuclease mapping bind to similar chromosomal bands, this would indicate that either they are part of the same gene, or that they are two genes that are closely linked on the chromosome. In either case, there are several ways to clone out these larger gene segments for further study. One technique is to prepare a cosmid library of group B Streptococcus and screen for hybridization with one of the probes of interest. When a clone is obtained that contains two or more genes of interest it could be endonuclease mapped and studied for the expression of protective antigens as described for the previously described clones.

The identification of the above-described clones permits their DNA sequences to be determined. If the clones are on the pUX12 plasmid, it is possible to use double stranded DNA sequencing with reverse transcriptase to sequence from oligonucleotide primers prepared to the polylinker. This technique was used earlier in characterizing the pUX12 plasmid and is a rapid way to sequence multiple additional oligonucleotide primers to sequence a gene that is larger than 600 base pairs. Therefore, the DNA sequencing for the C protein genes is preferably performed by subcloning into an M13, single stranded DNA sequencing system (Ausubel, F.M., et al., Current Topics in Molecular Biology (1987)).

The elucidation of the DNA sequences of the C proteins provides substantial information regarding the structure, function and regulation of the genes and their protein products. As discussed earlier, the heterogeneity in the sizes of C proteins isolated by many investigators and their apparent antigenic diversity suggests the possibility of either a gene family, or a post-transcriptional mechanism for modifying the protein products of the C protein genes (Ferrieri, P., et al., Infect. Immun. 27:1023-1032 (1980)). The M protein of group A Streptococcus was discussed earlier as an example of this phenomenon (Scott, J.R., et al., Proc. Natl. Acad. Sci. USA 82:1822-1826 (1985)). Although the DNA sequence of M protein shows no homology with group B Streptococcus chromosomal DNA by hybridization, there may be structural homologies between their DNA sequences (Hollingshead, S.K., et al., J. Biol. Chem. 261:1677-1686 (1986), Scott, J.R., et al. Proc. Natl. Acad. Sci. USA 82:1822-1826 (1985), Scott, J.R., et al., Infec. and Immun. 52:609-612 (1986)). The DNA sequences of the C proteins are preferably compared with a library of known DNA sequences. In addition, the amino acid sequences derived from the DNA sequences are compared with a library of known amino acid sequences.

### EXAMPLE 9

### PREVALENCE OF THE C PROTEIN GENES

To determine the prevalence of the C protein genes, chromosomal DNA from clinical and laboratory isolates of the various serotypes of group B Streptococcus are probed on genomic Southern blots with the C protein genes. In addition, comparison of the phenotypic expression as determined by precipitin techniques with genetic composition as shown by DNA-DNA hybridization is preformed in order to provide information regarding the regulation of expression of the C protein genes. The probes of the C protein genes are used to screen chromosomal DNA from other types of Streptococcus, and other bacterial pathogens.

Probes are prepared and labelled from the C protein genes of isolates of group B Streptococcus which includes most of the original typing strains used by Lancefield (Lancefield, R.C., et al., J. Exp. Med. 142:165-179 (1975)). Colony blots of the 24 clinical and laboratory isolates of group B Streptococcus are screened using the microtiter technique described above. The ability of the various strains to hybridize to the C protein genes is then compared with the phenotypic characteristics of these organisms in binding to typing antisera directed against the C proteins. In this manner, it is possible to determine what strains carry any or all of the C protein genes, and whether some strains carry silent or cryptic copies of these genes.

Those strains that hybridize to the C protein gene probes on colony blots are then screened using genomic Southern blots to determine the size, structure and location of their C protein genes. Chromosomal DNA isolated from the strains of group B Streptococcus that show binding on the colony blots is digested with restriction endonucleases, run on an agarose gel and blotted onto nitrocellulose. These Southern blots are probed with probes of the C protein genes. In this manner, it is possible to determine if there are differences in the location and size of these genes in the different serotypes of group B Streptococcus and to compare clinical (i.e. potentially virulent) isolates with laboratory strains (and with those which colonize clinically but are not associated with infection).

The C protein gene probes are also preferably used to screen other streptococcal strains and a variety of pathogenic bacteria. Streptococcal strains are known to share other proteins associated with virulence including the M and G proteins (Fahnestock, S.R., et al., J. Bact. 167(3):870-880 (1986), Heath, D.G., et al., Infec. and Immun. 55:1233-1238 (1987), Scott, J.R., et al., Infec. and Immun. 52:609-612 (1986), Walker, J.A., et al., Infec. and Immun. 55:1184-1189 (1987)). The strains to be tested are first screened using colony blots to determine whether they have any homologous sequences with the C protein genes probes. Genomic Southern blots are then prepared with the chromosomal DNA of the bacterial strains that test positive on the colony blots. These blots are then probed with the C protein genes to localize and define the areas of homology, such as a region of a C protein which serves as a membrane anchor, binds to the Fc region of immunoglobulins, or shares regions of homology with other genes with similar functions in other bacteria.

### EXAMPLE 10

### MODIFICATION OF THE C PROTEIN GENES IN GROUP B STREPTOCOCCUS

A number of potential virulence associated properties have been ascribed to the C proteins including resistance opsonization and inhibition of intracellular killing following phagocytosis (Payne, N.R, et al., J. Infec. Dis. 151:672-681 (1985), Payne, N.R., et al., Infect. and Immun. 55:1243-1251 (1987)). To better understand the roles of the C proteins in virulence, isogeneic strains are constructed in which the C protein genes are individually mutated. These strains will be tested for virulence in the neonatal rat model (Zeligs, B.J., et al., Infec. and Immun. 37:255-263 (1982). Two methods may be utilized to create isogeneic strains to evaluate the role of the C proteins in the virulence of group B Streptococcus. Preferably, tranposon mutagenesis with the self-conjugative transposon tn916 may be employed. Alternatively, site-directed mutagenesis may be used. The lack of efficient methods for genetic manipulation in group B Streptococcus necessitates the development of new genetic techniques to modify genes in group B Streptococcus and create isogeneic strains for studying virulence (Lopilato, J., et al., Mol. Gen. Genet. 205:285-290 (1986)).

Transposon insertional mutagenesis is a commonly used technique for constructing isogeneic strains that differ in the expression of antigens associated with virulence, and its use in group B Streptococcus is well described (Caparon, M.G., et al., Proc. Natl. Acad. Sci. USA 84:8677-8681 (1987), Rubens, C.E., et al., Proc. Natl. Acad. Sci USA 84:7208-7212 (1987), Wanger, A.R, Res. Vet. Sci. 38:202-208 (1985), Weiser, J.N., Trans Assoc. Amer. Phys. 98:384-391 (1985)). Rubens, et al. have demonstrated the utility of Tn916 is studies of the group B Streptococcus capsule (Rubens, C.E., Proc. Natl. Acad. Sci. USA 84:7208-7212 (1987). The self-conjugating transposon TN916 may be made from Streptococcus faecalis into group B Streptococcus as previously described (Wanger, A.R., Res. Vet. Sci. 38:202-208 (1985) which reference is incorporated herein by reference). Strains are selected for the acquisition of an antibiotic resistance marker, and screened on colony blots for the absence of expression of the C proteins as detected by the specific antisera prepared as described above. Isolates that do not appear to express the C proteins can be further mapped using genomic Southern blots to localize the insertion within the C protein genes. The original Tn916 strain carried tet^{R}; however, an erythromycin resistance marker has recently been cloned into Tn916 (Rubens, C.E., et al., Plasmid 20:137-142 (1988)). It is necessary to show that, following mutagenesis with Tn916, only one copy of the transposon is carried by the mutant strain and that the transposon is localized within the C protein gene.

The application of these techniques to deleting the C protein genes in group B Streptococcus is straightforward, unless a C protein genes is essential to the survival of group B Streptococcus. However, strains of group B Streptococcus have been described that lack any detectable C protein and it is unusual for a bacterial virulence determinant to be an essential gene for survival in vitro. An additional use of Tn916 that will be explored is the identification of potential regulatory elements of the C protein genes.

In the event that specific defined mutations are desired or if the C protein gene is essential for the viability of group B Streptococcus, techniques of site-directed mutagenesis may be employed (for example to produce conditional mutants). Site-directed mutagenesis may thus be used for the genetic analysis of group B Streptococcus proteins. One problem that has delayed the development of these techniques in group B Streptococcus is the difficulty encountered in transforming group B Streptococcus. Electroporation has proven valuable in introducing DNA into bacteria that are otherwise difficult to transform (Shigekawa, K., et al., Biotech 6:742-751 (1988)). Conditions for transforming group B Streptococcus utilizing electroporation may be utilized to surmount this obstacle. It is thus possible to do site directed mutagenesis, to evaluate complementation, and to introduce C protein genes into group B Streptococcus strains that do not express the C proteins. Any of several approaches may be utilized to insert native or mutated C protein genes into strains of group B Streptococcus. For example, a drug resistance marker may be inserted within the C protein gene clones in pUX12. A drug resistance marker that can be expressed in group B Streptococcus, but that is not normally present, is preferred. This modified pUX12 protein clone is transformed into group B Streptococcus using electroporation (Shigekawa, K., et al., BioTech 6:742-751 (1988)). Since the pUX12 plasmid cannot replicate in group B Streptococcus, those strains that acquire the drug resistance phenotype would likely do so by homologous recombination between the C protein gene on the host GB chromosome and the mutated C protein carried on the pUX12 plasmid. The mutants are screened as described above. If there are no homologous sequences in the recipient strain, it is possible to construct a vector with the C protein gene inserted within a known streptococcal gene, i.e., a native drug resistance marker gene from group B Streptococcus. Following electroporation, such a plasmid construct would integrate into the chromosome via homologous recombination.

Alternatively, modified C protein genes could be introduced into the group B Streptococcus chromosome by inserting the genes into the self-conjugating transposon Tn916 and introducing the modified transposons via mating from Streptococcus faecalis. This technique was used to successfully modify Tn916 with an erythromycin gene and insert this gene into the chromosome of group B Streptococcus (Rubens, C.E., et al., Plasmid 20:137-142 (1988)). It is necessary to show that, following mutagenesis with Tn916, only one copy of the transposon is carried by the mutant strain and that the transposon is localized within the C protein gene.

### EXAMPLE 11

### EVALUATION OF THE ROLE OF THE C PROTEINS IN VIRULENCE OF GROUP B STREPTOCOCCUS

Previous studies that compared strains of group B Streptococcus that do and do not carry C proteins involved isolates that were not known to be isogeneic (Ferrieri, P. et al., Rev. Inf. Dis. 10(2): 1004-1071 (1988)). Therefore, it was not possible to determine whether the differences in virulence observed are related to the C proteins or to some other virulence determinant. The construction of isogeneic strains having either intact C protein genes or C protein gene deletions permit a characterization of the role of the C protein in vurulence. The strains are preferably tested in the neonatal rat model for virulence and in the mouse protection model for their immunological properties. A second important test of virulence is the ability of a gene to restore virulence through reversion of allelic replacement in a mutant strain. By inserting the C protein genes into group B Streptococcus strains that either do not carry the gene or which carry inactivated C protein genes, it is possible to determine the effect of the C protein by examining the virulence of the resulting construct in the above animal models.

Isogeneic strains of group B Streptococcus in which the C protein genes are individually mutated may be created using either transposon mutagenesis or site-directed mutagenesis. Such strains are preferably characterized on genomic Southern blots to determine that only a single insertion is present on the chromosome. The location of these insertions may be ascertained using the fine structure genetic mapping techniques discussed above. The isogeneic strains are then tested for virulence in the neonatal rat model (Zeligs, B.J., et al., Infec. and Immun. 37:255-263 (1982)).

Transposon mutagenesis permits the identification of genes involved in regulating the expression of the C proteins. For example, strains carrying the wild type C protein genes which are found to no longer express C proteins following transposon mutagenesis and in which transposon is not located within the C protein structural gene, carry mutations in sequences involved in the regulation of expression of the C protein genes. This approach was used successfully in characterizing the mry locus in group A Streptococcus that is involved in regulation of the M protein (Caparon, M.G., et al., Proc. Natl. Acad. Sci. USA 84:8677-8681 (1987), Robbins, J.C., et al., J. Bacteriol 169:5633-5640 (1987)). Such methods may also be used to produce strains which overexpresses the C proteins, or which produce C proteins of altered virulence or immunity.

### EXAMPLE 12

### LOCALIZATION OF THE C PROTEINS ON GROUP B STREPTOCOCCUS AND EVALUATION OF THEIR ABILITY TO BIND TO IMMUNOGLOBULINS

Lancefield and others have shown that antibody to the C proteins binds to the outer membrane of group B Streptococcus (Lancefield, R.C., et al., J. Exp. Med. 142:165-179 (1975), Wagner, B., et al., J. Gen Microbiol. 118:95-105 (1980)). This suggests that the C protein is an outer membrane protein. C proteins can also be isolated from the supernatants of cultures of group B Streptococcus, indicating that these proteins may be either secreted by group B Streptococcus or lost at a high rate from the cell surface. The DNA and protein sequences derived from the C protein genes are valuable in determining the structure and function of the C proteins. One potential virulence determinant commonly described for the C proteins is the ability to bind to the immunoglobulin, IgA (Ferrieri, P. et al. Rev. Inf. Dis. 10(2):1004-1071 (1988), Russell-Jones, G.J., et al., J. Exp. Med. 160:1467-1475 (1984)).

Immuno-electron microscopy has been utilized to localize cell surface determinants that are detected by specific antibody. Antisera raised against the C protein clones of group B Streptococcus is incubated with group B Streptococcus strains that carry the C proteins. Ferritin-conjugated goat anti-rabbit IgG is used to detect the antigen on the cell surface as previously described (Rubens, C.E., et al., Proc. Natl. Acad. Sci. USA 84:7208-7212 (1987), Wagner B., et al.. J. Gen. Microbiol. 118:95-105 (1980)).

A simple determination of the ability of C proteins to bind to immunoglobulins can be assessed using Western blots. Cellular extracts of both the E. coli clones containing the C protein genes and of group B Streptococcus strains that carry the C proteins can be run on SDS-PAGE and blotted onto nitrocellulose. Controls include extracts of E coli. carrying the wild type pUX12 plasmid, strains of group B Streptococcus that do not carry the C protein genes, and isogeneic group B Streptococcus strains in which the C protein genes have been inactivated. The Western blots can be probed individually with labelled immunoglobulins, e.g., IgG, IgM, IgA, and their components, e.g., the Fc or F(ab)₂ fragments (Heath, D.G., et al., Infect. and Immun. 55:1233-1238 (1987), Russell-Jones, G.J., et al., J. Exp. Med. 160:1467-1475 (1984)). The immunoglobulins are preferably iodinated using either iodogen or chloramine T.

A more specific way to measure the ability of the C proteins to bind to immunoglobulins and their components involves purifying the C proteins and using them directly in a binding assay (Fahnestock, S.R., et al., J Bact. 167(3):870-880 (1986), Heath, D.G., et al., Infect. and Immun. 55:1233-1238 (1987)). Using the protein sequence, one can purify the C protein. In addition, since it is possible to express the C protein genes in E coli., one may construct E. coli strains that overproduce the C proteins and thereby obtain larger amounts of C proteins for purification.

Although the foregoing refers to particular preferred embodiments, it will be understood that the present invention is not so limited. It will occur to those ordinarily skilled in the art that various modifications may be made to the disclosed embodiments and that such modifications are intended to be within the scope of the present invention.

### EXAMPLE 13

### USE OF THE CLONED C PROTEIN ANTIGENS OF GROUP B STREPTOCOCCUS IN A CONJUGATE VACCINE

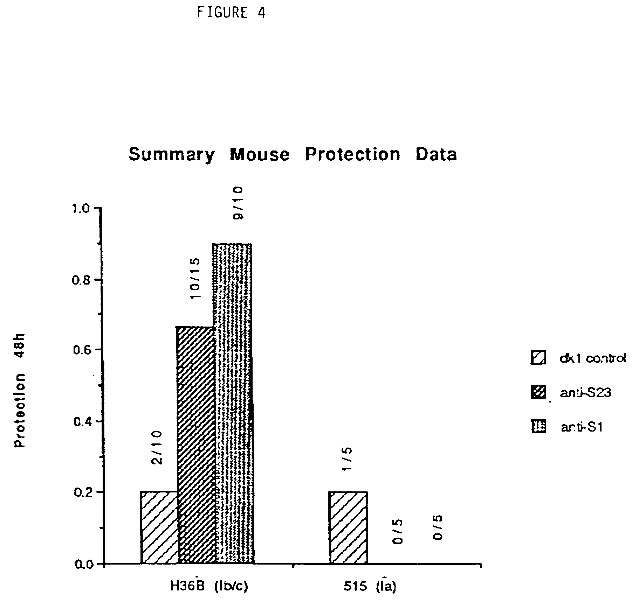

The above-described protective C protein antigens of group B Streptococcus were tested for their potential in a conjugate vaccine. To assess this potential, cellular extracts of E. coli containing pJMS1 or pJMS23 were prepared as decribed above, and used to immunize rabbits. The resulting antisera was tested in the mouse lethality model for its ability to protect mice from infection by the group B Streptococcus strain H36B. Strain H36B carries the C protein of group B Streptococcus. As a control, the ability of the antisera to protect the mice against infection by Streptococcus strain 515 (which does not carry the C protein) was determined. The results of this experiment are shown in Figure 4.

## Claims

1. A conjugate vaccine capable of conferring host immunity to an infection by group B Streptococcus wherein said vaccine comprises:
a) a capsular polysaccharide conjugated to
b) at least one recombinant C protein of said group B Streptococcus,
wherein said protein is selected from the group consisting of a recombinant S1 protein and a recombinant S23 protein, wherein said S1 protein is encoded by plasmid pJMS1 (ATCC #40659) and wherein said S23 protein is encoded by plasmid pJMS23 (ATCC #40660) and wherein said vaccine is substantially free of non-C streptococcal proteins and is in a pharmacologically acceptable composition.

2. A recombinant molecule which is the plasmid pJMS23 (ATCC #40660) comprising a gene sequence which encodes S23 protein of a group B Streptococcus.

3. The recombinant molecule of claim 2, wherein said molecule is capable of expressing said protein in a bacteria.

4. The recombinant molecule of claim 2, wherein said molecule is capable of expressing said C protein in a bacteria.

5. The recombinant molecule of claim 3, wherein said bacteria is the bacteria Escherichia coli.

6. Use of
a) a capsular polysaccharide characteristic of group B Streptococcus conjugated to
b) at least on recombinant C protein of said group B Streptococcus,
wherein said protein is selected from the group consisting of a recombinant S1 protein and a recombinant S23 protein, wherein said S1 protein is encoded by plasmid pJMS1 (ATCC #40659) and wherein said S23 protein is encoded by plasmid pJMS23 (ATCC #40660) and wherein the conjugate of capsular polysaccharide and recombinant C protein is substantially free of non-C streptococcal proteins and is in a pharmacologically acceptable composition for the production of a conjugate vaccine capable of conferring host immunity to an infection caused by a group B Streptococcus for administering an effective amount of said vaccine to an individual, suspected of being at risk for such an infection.

7. The use of claim 6 for the production of a conjugate vaccine capable of conferring immunity to an infection caused by a group B Streptococcus to an unborn offspring of a female for administering an effective amount of said vaccine to a pregnant female.

8. The use of claim 6 for the production of antisera elicited from the exposure of an individual to the conjugate vaccine for administering to an individual suspected being at risk for an infection caused by a group B Streptococcus.

9. The antisera of claim 8 wherein said antisera is labeled with a toxin.

## Patentansprüche

1. Ein konjugierter Impfstoff, der fähig ist, Wirtsimmunität gegenüber Streptokokken der Gruppe B zu verleihen, wobei dieser Impfstoff umfaßt:
a) ein Kapselpolysaccharid konjugiert mit
b) mindestens einem rekombinanten C-Protein von den Streptokokken der Gruppe B,
wobei das Protein aus jener Gruppe ausgewählt ist, welche ein rekombinantes S1 Protein und ein rekombinantes S23 Protein enthält, wobei das S1 Protein vom Plasmid pJMS1 (ATCC #40659) codiert wird und das S23 Protein vom Plasmid pJMS23 (ATCC #40660) codiert wird und wobei der Impfstoff im wesentlichen frei von nicht-C Streptokokkenproteinen ist und in einer pharmakologisch annehmbaren Zusammensetzung vorliegt.

2. Ein rekombinantes Molekül, bei dem es sich um das Plasmid pJMS23 (ATCC #40660) handelt, welches eine Gensequenz enthält, die für das S23 Protein eines Streptokokkus der Gruppe B codiert.

3. Das rekombinante Molekül nach Anspruch 2, wobei das Molekül befähigt ist, das Protein in Bakterien zu exprimieren.

4. Das rekombinante Molekül nach Anspruch 2, wobei das Molekül befähigt ist, das C-Protein in Bakterien zu exprimieren.

5. Das rekombinante Molekül nach Anspruch 3, wobei das Bakterium Escherichia coli ist.

6. Die Verwendung von
a) einem Kapselpolysaccharid, das charakteristisch für Streptokokken der Gruppe B ist, konjugiert mit
b) mindestens einem rekombinanten C-Protein von den Streptokokken der Gruppe B,
wobei das Protein aus jener Gruppe ausgewählt ist, welche ein rekombinantes S1 Protein und ein rekombinantes S23 Protein enthält, wobei das S1 Protein vom Plasmid pJMS1 (ATCC #40659) codiert wird und das S23 Protein vom Plasmid pJMS23 (ATCC #40660) codiert wird und wobei das Konjugat aus Kapselpolysaccharid und rekombinantem C-Protein im wesentlichen frei von nicht-C Streptokokkenproteinen ist und in einer pharmakologisch annehmbaren Zusammensetzung für die Herstellung eines konjugierten Impfstoffs vorliegt, der fähig ist, Wirtsimmunität gegenüber einer Infektion durch Streptokokken der Gruppe B zu vermitteln, zur Verabreichung einer wirksamen Menge dieses Impfstoffes an ein Individuum, bei welchem man ein Risiko für eine solche Infektion vermutet.

7. Die Verwendung nach Anspruch 6, zur Herstellung eines konjugierten Impfstoffes, der fähig ist, Immunität gegenüber einer Infektion durch Streptokokken der Gruppe B bei ungeborenen Kindern zu vermitteln, wobei der schwangeren Frau eine wirksame Menge dieses Impfstoffes verabreicht wird.

8. Die Verwendung nach Anspruch 6, zur Herstellung eines Antiserums, das gebildet wird, wenn ein Individuum dem konjugierten Impfstoff ausgesetzt wird, zur Verabreichung an Personen, bei denen ein Risiko einer Infektion mit Streptokokken der Gruppe B vermutet wird.

9. Das Antiserum nach Anspruch 8, wobei das Antiserum mit einem Toxin markiert ist.

## Revendications

1. Vaccin conjugué capable d'immuniser l'hôte contre une infection par Streptocoque du groupe B, ledit vaccin comprenant :
a) un polysaccharide capsulaire conjugué à
b) au moins une protéine recombinante C dudit Streptocoque du groupe B, ladite protéine étant choisie dans le groupe constitué d'une protéine recombinante S1 et d'une protéine recombinante S23, ladite protéine S1 étant codée par le plasmide pJMS1 (ATCC #40659) et ladite protéine S23 étant codée par le plasmide pJMS23 (ATCC #40660) et ledit vaccin étant pratiquement exempt de protéines streptococciques non-C et étant dans une préparation pharmacologiquement acceptable.

2. Molécule recombinante qui est le plasmide pJMS23 (ATCC #40660), comprenant une séquence de ge'ne qui code pour la protéine S23 d'un Streptocoque du groupe B.

3. Molécule recombinante selon la revendication 2, ladite molécule étant capable d'exprimer ladite protéine dans une bactérie.

4. Molécule recombinante selon la revendication 2, ladite molécule étant capable d'exprimer ladite protéine C dans une bactérie.

5. Molécule recombinante selon la revendication 3, ladite bactérie étant la bactérie Escherichia coli.

6. Utilisation de
a) un polysaccharide capsulaire caractéristique du Streptocoque du groupe B conjugué à
b) au moins une protéine recombinante C dudit Streptocoque du groupe B, ladite protéine étant choisie dans le groupe constitué d'une protéine recombinante S1 et d'une protéine recombinante S23, ladite protéine S1 étant codée par le plasmide pJMS1 (ATCC #40659) et ladite protéine S23 étant codée par le plasmide pJMS23 (ATCC #40660) et le conjugué du polysaccharide capsulaire et de la protéine recombinante C étant pratiquement exempt de protéines streptococciques non-C et étant dans une préparation pharmacologiquement acceptable pour la production d'un vaccin conjugué capable d'immuniser l'hôte contre une infection causée par un Streptocoque du groupe B, destiné à l'administration d'une quantité efficace dudit vaccin à un individu suspecté de risquer une telle infection.

7. Utilisation selon la revendication 6 pour la production d'un vaccin conjugué, capable d'immuniser l'enfant à naître d'une femme contre une infection causée par un Streptocoque du groupe B, pour l'administration d'une quantité efficace dudit vaccin à une femme enceinte.

8. Utilisation selon la revendication 6 pour la production d'antiserum obtenu par l'exposition d'un individu au vaccin conjugué destiné à être administré à un individu suspecté du risque d'une infection causée par un Streptocoque du groupe B.

9. Antisérum selon la revendication 8, ledit antisérum étant marqué avec une toxine.
